(19) **Europäisches Patentamt**
**European Patent Office**
**Office européen des brevets**

(11) **EP 2 790 658 B1**

(12) **EUROPEAN PATENT SPECIFICATION**

(45) Date of publication and mention
of the grant of the patent:
**16.08.2017   Bulletin 2017/33**

(51) Int Cl.:
*A61K 8/49* *(2006.01)*        *A61K 8/66* *(2006.01)*
*A61Q 5/00* *(2006.01)*        *A61K 8/22* *(2006.01)*
*A61Q 5/02* *(2006.01)*        *A61Q 5/10* *(2006.01)*

(21) Application number: **12798756.8**

(22) Date of filing: **11.12.2012**

(86) International application number:
**PCT/EP2012/075121**

(87) International publication number:
**WO 2013/087644 (20.06.2013 Gazette 2013/25)**

(54) **METHOD OF STRENGTHENING HAIR FIBRES**

VERFAHREN ZUR STÄRKUNG VON HAARFASERN

PROCÉDÉ DE RENFORCEMENT DE LA RÉSISTANCE DE FIBRES CAPILLAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.12.2011   EP 11192986**

(43) Date of publication of application:
**22.10.2014   Bulletin 2014/43**

(73) Proprietors:
• **Unilever PLC**
**London, Greater London EC4Y 0DY (GB)**
Designated Contracting States:
**CY GB IE MT**
• **Unilever N.V.**
**3013 AL Rotterdam (NL)**
Designated Contracting States:
**AL AT BE BG CH CZ DE DK EE ES FI FR GR HR HU IS IT LI LT LU LV MC MK NL NO PL PT RO RS SE SI SK SM TR**

(72) Inventors:
• **BHOGAL, Ranjit, Kaur**
**Bedford**
**Bedfordshire MK44 1LQ (GB)**
• **CASEY, John**
**Bedford**
**Bedfordshire MK44 1LQ (GB)**
• **D'AGOSTINO, Eleanor, Margaret**
**Bedford**
**Bedfordshire MK44 1LQ (GB)**

(74) Representative: **Warner, Guy Jonathan**
**Unilver PLC**
**Unilever Patent Group**
**Colworth House**
**Sharnbrook**
**Bedford, Bedfordshire MK44 1LQ (GB)**

(56) References cited:
**EP-A1- 1 142 563          WO-A1-99/15137**
**US-A1- 2004 261 198**

• **MOHAMMED HOSNY ET AL: "Novel Oxidations of (+)-Catechin by Horseradish Peroxidase and Laccase", JOURNAL OF AGRICULTURAL AND FOOD CHEMISTRY, vol. 50, no. 20, 1 September 2002 (2002-09-01), pages 5539-5545, XP055028492, ISSN: 0021-8561, DOI: 10.1021/jf020503j**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The invention relates to a method of repairing hair fibres using flavonoids, hydrogen peroxide and a peroxidase enzyme.

Hair can be damaged in a number of ways including exposure to heat, bleaching, use of shampoos and styling products, brushing and combing, and exposure to the environment, for example ultra-violet light. Existing treatments designed to repair damaged hair make use of surface active materials that mask the problem rather than actually repairing the hair. These materials modify fibre feel by changing consumer perceivable fibre sensory cues such as smoothness, may change some measureable physical properties such as hydrophobicity and hydrophilicity, but do not change other physical characteristics such as fibre stiffness, strength or structural integrity.

US 5 770 418 (Novo Nordisk A/S) discloses the dyeing effect on blond European hair tresses at 30 °C for 60 minutes of *Polyporus pinsitus* laccase or hydrogen peroxide with the following dye precursors: phenylene diamine (PPD); toluylene diamine; chloro-p-phenylenediamine; p-aminophenol; o-aminophenol; 3, 4-diaminotoluene; m-phenylene-diamine; 2, 4-diaminoanisole; α-naphthol; hydroquinone; pyrocatechol; resorcinol; and 4-chlororesorcinol.

US 2004/0261198 (Henkel Corporation) discloses a method for modifying keratin fibres, in particular for restructuring and finishing by positively influencing the fibre properties, in particular the strength, porosity, elasticity, colour retention and volume of the fibres, by polymerising suitable polymerisable substrates at the fibre by using a polyphenol oxidase. The compositions should be free of peroxides or hydroperoxides. Swelling of the hair in the wet state is described as a measure of hair damage. Suitable substrates are phenolic compounds substituted by 1 to 5 groups, an example of which includes catechin; aromatic amines; enolic ompounds; and enaminic compounds. The invention was exemplified with a combination of green tea powder, a polyphenol oxidase from the fungus Myceliophtora, and methyl syringate as mediator, at pH 6.5-7.5 and at 32 °C on bleached hair. Significant increases in the stress and work values in the plastic range for wet individual hair fibres were observed demonstrating restructuring of the bleached hair by the green tea and the laccase enzyme.

WO 2010/130526 (Henkel AG & Co. KGAA) discloses a cosmetic hair treatment agent free from hydrogen peroxide, the agent comprising, in a cosmetic carrier, a surface-active agent and an acetylpyridinium derivative of a given formula for improving the general condition of hair fibres and increasing the elasticity of the hair. The agent may additionally include a plant extract. Green tea is disclosed as an example of such a plant extract.

WO 2010/130510 (Henkel AG & Co. KGAA) discloses a cosmetic hair shaping product comprising, in a cosmetic carrier, a hair strengthening and/or film-forming polymer, and an acetylpyridinium derivative of a given formula for reducing hair damage in the interior of the hair and for increasing the elasticity of the hair. The product may additionally include a plant extract. Green tea is disclosed as an example of such a plant extract.

**SUMMARY OF THE INVENTION**

In a first aspect a method of strengthening hair fibres is provided, the method comprising the step of applying a hair composition comprising:

(a) A flavonoid;
(b) A hydrogen peroxide generator or hydrogen peroxide; and
(c) A peroxidase;

wherein the composition has a pH of 3 to 9, preferably 4.5 to 7, more preferably less than or equal to 6,
wherein the $\Delta E$ value of the hair fibres before and after application of the hair composition is at least +5.

**SUMMARY OF THE FIGURES**

The invention is exemplified with reference to the following figures in which:

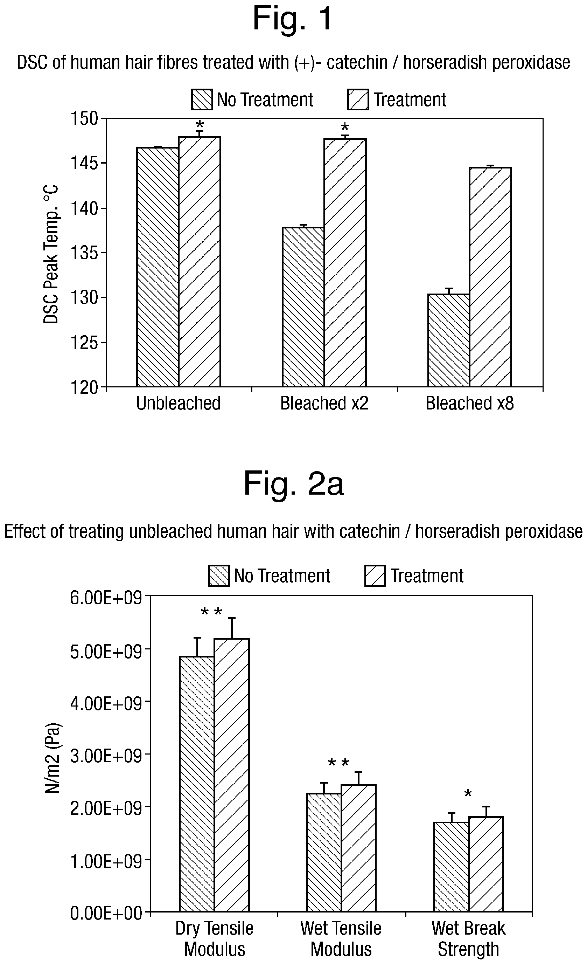

Figure 1 shows denaturation peak temperature (°C) for (+)-catechin, horseradish peroxidase, and hydrogen peroxide combination on natural white hair unbleached, and bleached x2 and x8 (all differences were significant ($p =< 0.05$) except * unbleached treated vs bleached treated x 2 (Student's t-test at 95 % confidence limits));

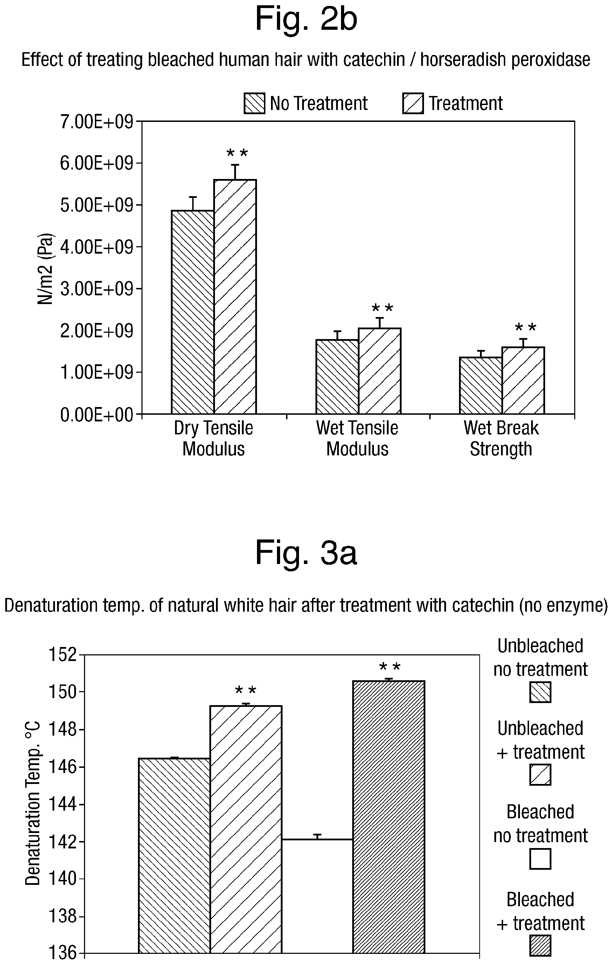

Figures 2a and 2b show dry and wet tensile moduli, and wet break strength test ($N/m^2$) on unbleached (2a) and bleached x2 (2b) natural white hair after treatment with (+)- catechin, horseradish peroxidase, and hydrogen peroxide combination (* $p = < 0.01$ and ** $p = < 0.001$ (Student's t-test at 95 % confidence limits));

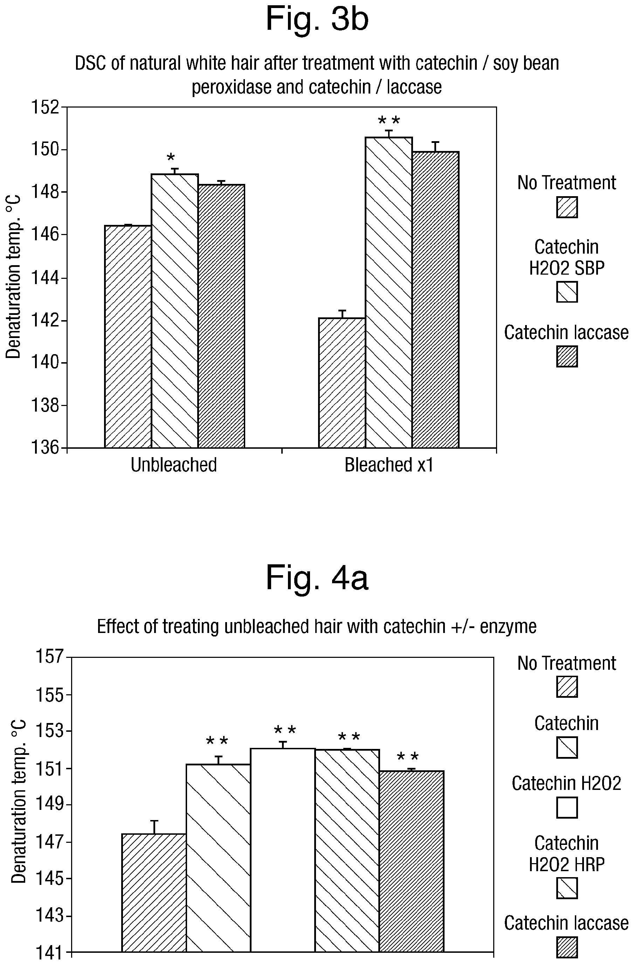

Figures 3a and 3b show denaturation peak temperature (°C) for (+)-catechin, soy bean peroxidase, and hydrogen peroxide combination (3b); (+)-catechin and laccase combination (3b); and (+)-catechin and hydrogen peroxide combination (3a); on natural white hair unbleached, and bleached x1 (* (+)-catechin / laccase p = 0.0029 (figure 3b), ** (+)-catechin / laccase p = 0.0072 (figure 3b), ** untreated control p < 0.0001 (figure 3a) (standard errors at 95 % confidence limits are provided));

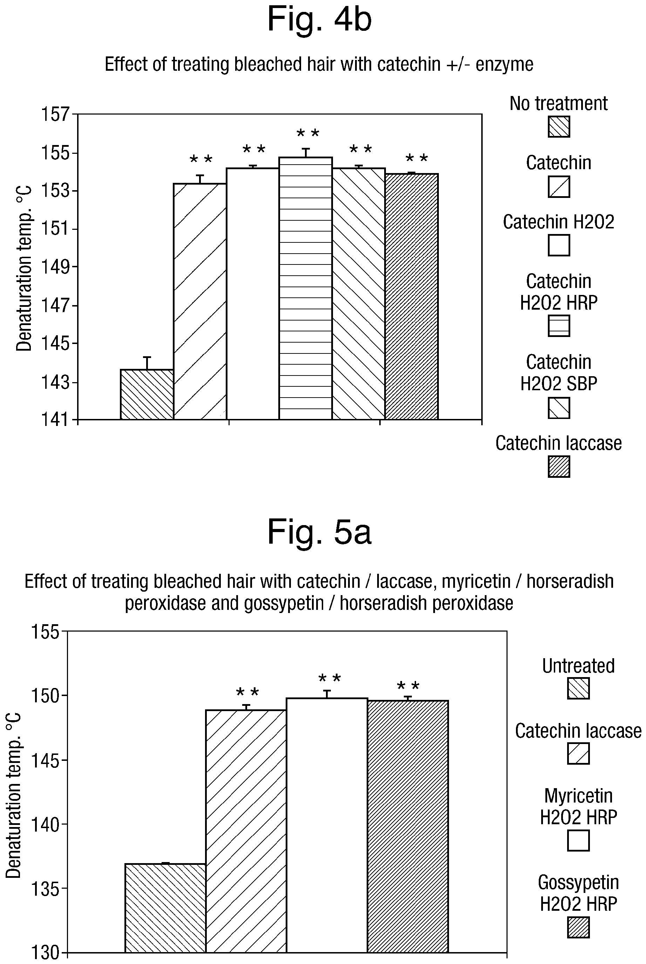

Figure 4a shows denaturation peak temperature (°C) for (+)-catechin, horseradish peroxidase, and hydrogen peroxide combination; (+)-catechin and laccase combination; (+)-catechin and hydrogen peroxide combination; and (+)-catechin on dark brown hair unbleached. (** untreated control p < 0.01 for standard error at 95 % confidence limits), whilst figure 4b shows denaturation peak temperature (°C) for (+)-catechin, horseradish peroxidase, and hydrogen peroxide combination; (+)-catechin, soy bean peroxidase, and hydrogen peroxide combination; (+)-catechin and laccase combination; (+)-catechin and hydrogen peroxide combination; and (+)-catechin on dark brown hair bleached x1 (** untreated control p < 0.0001 for standard error at 95 % confidence limits); and

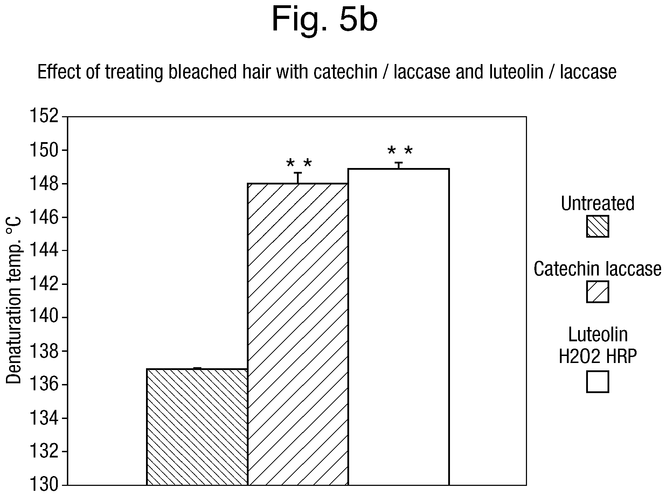

Figure 5a shows the denaturation peak temperature (°C) for (+)-catechin and laccase combination; myricetin, hydrogen peroxide and horseradish peroxidase combination; and gossypetin, hydrogen peroxide and horseradish peroxidase combination on double bleached natural white hair. (** versus untreated control p < 0.0001 for standard error at 95 % confidence limits)., whilst figure 5b shows the denaturation peak temperature (°C) for (+)-catechin and laccase combination; and luteolin, hydrogen peroxide and horseradish peroxidase combination on double bleached natural white hair (** versus untreated control p < 0.0001 for standard error at 95 % confidence limits).

## DETAILED DESCRIPTION OF THE INVENTION

This invention is based on the observation by the inventors that the thermomechanical and mechanical properties of damaged hair fibres can be improved by treatment with flavonoids, optionally in the presence of hydrogen peroxide, or a combination of hydrogen peroxide and a peroxidase enzyme.

Thus a method of strengthening hair fibres is provided, the method comprising the step of applying a hair composition comprising:

(a) A flavonoid; and
(b) A hydrogen peroxide generator or hydrogen peroxide; and
(c) A peroxidase;

wherein the composition has a pH of 3 to 9, preferably 4.5 to 7, more preferably less than or equal to 6, wherein the ΔE value of the hair fibres before and after application of the hair composition is at least +5.

In the alternative, use of a hair composition is provided, the composition comprising:

(a) A flavonoid; and
(b) A hydrogen peroxide generator or hydrogen peroxide; and
(c) A peroxidase;

wherein the composition has a pH of 3 to 9, preferably 4.5 to 7, more preferably less than or equal to 6, wherein the ΔE value of the hair fibres before and after application of the hair composition is at least +5, for strengthening hair fibres.

The flavonoid may be selected from the group consisting of flavones, isoflavones, flavans, isoflavans, flavanones, flavonols, flavan-3-ols, dihydroflavonol, flavanonols, anthocyanidins, proanthocyanidins, auron, chalcone, dihydrochalcone, flavonolignans, and derivatives thereof. Preferably the flavone is selected from the group consisting of luteolin and chrysin; the isoflavone is selected from the group consisting of daidzein, genistein and formononetin; the flavan is 4'-hydroxy-5,6-dimethoxyflavan; the isoflavan is glabridin or licoricidin; the flavanone is eriodictyol; the flavonol is selected from the group consisting of myricetin, kaempferol, gossypetin and quercetin; the flavan-3-ol is selected from the group consisting of catechin, theaflavin, gallocatechin, catechin gallate, gallocatechin gallate, epicatechin, epigallocatechin, epigallocatechin gallate and epigallocatechin gallate; the dihydroflavonol is taxifolin or aromadendrin; the anthocyanidin is selected from the group consisting of cyanidin, delphinidin, pelargonidin and malvidin; the aurone is sulphuretin; the chalcone is 2'-hydroxy-4-methoxy-chalcone; the dihydrochalcone is phloretin or phloridzin and the flavonolignan is silibinin or silichristin.

In one particular embodiment, the flavonoid is selected from the group consisting of silibinin, silandrin, 3,4-dihydroxyflavone, hesperidin (glycoside), naringin (naringenin glycoside), amentoflavone, rutin, eriodictyol-7-O-glucoside, quercitrin, kaempferol, pinostrobin and biochanin A.

In another particular embodiment, the flavonoid is selected from the group consisting of (+)-catechin, myricetin, gossypetin and luteolin

The peroxidase is preferably a non-animal haem peroxidase from class II (fungi) or class III (plants and algae). Examples include those obtained from the group consisting of Arabidopsis thaliana, horse radish, barley, peanut soybean, tobacco, and turnip (plants), Chlorophyta spirogyra (green algae), Arthromyces ramosus and Corprinus cinereus (fungi).

The hair colour composition preferably comprises 0.01 - 10, preferably 0.1 - 5 % w/w flavonoid; 0.0001 - 3 preferably 0.001 - 1, most preferably 0.01 - 1 % w/w hydrogen peroxide; and 0.0001 - 5, preferably 0.001 - 1 % w/w peroxidase.

When using a hydrogen peroxide generator, this comprises a hydrogen peroxide generating oxidase, a substrate and oxygen. The hydrogen peroxide generating oxidase may be selected from the group consisting of (S)-2-hydroxy acid oxidase, D-galactose oxidase, glucose oxidase, coniferyl alcohol oxidase, glycolate oxidase, hexose oxidase, oxalate oxidase, amino acid oxidase and L-galactonolactone oxidase and the respective substrate is selected from the group consisting of (S)-2-hydroxy acid, D-galactose, glucose, coniferyl alcohol, $\alpha$-hydroxy acids, D-glucose, oxalic acid, amino acid and L-galactono-1,4-lactone. Specifically the following combinations may be used: (S)-2-hydroxy acid with (S)-2-hydroxy acid oxidase; D-galactose with D-galactose oxidase; glucose with glucose oxidase; coniferyl alcohol with coniferyl alcohol oxidase; $\alpha$-hydroxy acids with glycolate oxidase; D-glucose with hexose oxidase; oxalic acid with oxalate oxidase; and L-galactono-1,4-lactone with L-galactonolactone oxidase; amino acid oxidase with amino acids; all in the presence of oxygen.

The hair colour composition may comprise 0.0001 - 5, preferably 0.001 - 1 % w/w hydrogen peroxide generating oxidase; and 0.01 - 10 preferably 0.1 - 5 % w/w substrate.

Optionally the hair colour composition comprises a metal ion suitable for coordinating to the flavonoid or oxidative product of the flavonoid, the metal ion being preferably selected from the group consisting of iron (II), iron (III), copper (I), copper (II), copper (III) and aluminium (III). When present, the hair colour composition may comprise 0.0001 - 2, preferably 0.001 - 0.1 % w/w metal ion.

The hair composition may take the form of a shampoo or hair conditioning composition, or a 2-in-1 conditioning shampoo composition.

### 1.0 Shampoo Compositions

Shampoo compositions will nearly always comprise a cleansing surfactant component in an aqueous base.

### 1.1 Cleansing Surfactant

The cleansing surfactant may consist of a single surfactant, usually an anionic surfactant (to provide foam) such as sodium lauryl ether sulphate, or more commonly a mixture of sodium lauryl ether sulphate with a co-surfactant to provide mildness. The most preferred co-surfactant is cocoamidopropyl betaine.

The total amount of surfactant (including any co-surfactant, and/or any emulsifier) in a shampoo composition may be from 1 to 50., preferably from 2 to 40, more preferably from 10 to 25 % w/w. Compositions comprising more than 25 % w/w cleansing surfactant are commonly considered concentrated shampoos.

Examples of suitable anionic cleansing surfactants are the alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphates, alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in shampoo compositions of the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecylbenzene sulphonate, triethanolamine dodecylbenzene sulphonate, sodium cocoyl isethionate, sodium lauryl isethionate, lauryl ether carboxylic acid and sodium N-lauryl sarcosinate.

Preferred anionic surfactants are the alkyl sulfates and alkyl ether sulfates. These materials have the respective formulae $R2OSO_3M$ and $R1O(C_2H_4O)_xSO_3M$, wherein R2 is alkyl or alkenyl of from 8 to 18 carbon atoms, x is an integer having a value of from about 1 to about 10, and M is a cation such as ammonium, alkanolamines, such as triethanolamine, monovalent metals, such as sodium and potassium, and polyvalent metal cations, such as magnesium, and calcium. Most preferably R2 has 12 to 14 carbon atoms, in a linear rather than branched chain.

Preferred anionic cleansing surfactants are selected from sodium lauryl sulphate and sodium lauryl ether sulphate(n)EO, (where n is from 1 to 3); more preferably sodium lauryl ether sulphate(n)EO, (where n is from 1 to 3); most preferably sodium lauryl ether sulphate(n)EO where n=1.

Preferably the level of alkyl ether sulphate is from 0.5 to 25, more preferably from 3 to 18, most preferably from 6 to 15 % w/w of the composition.

The total amount of anionic cleansing surfactant in shampoo compositions of the invention generally ranges from 0.5 to 45, more preferably from 1.5 to 20 % w/w of the composition.

## 1.2 Nonionic Surfactant

Shampoo compositions of the invention may contain non-ionic surfactant. Most preferably non-ionic surfactants are present in the range 0 to 5 % w/w of the composition.

Nonionic surfactants that can be included in shampoo compositions of the invention include condensation products of aliphatic (C8 - C18) primary or secondary linear or branched chain alcohols or phenols with alkylene oxides, usually ethylene oxide and generally having from 6 to 30 ethylene oxide groups. Alkyl ethoxylates are particularly preferred. Most preferred are alkyl ethoxylates having the formula $R-(OCH_2CH_2)nOH$, where R is an alkyl chain of C12 to C15, and n is 5 to 9.

Other suitable nonionic surfactants include mono- or di-alkyl alkanolamides. Examples include coco mono- or di-ethanolamide and coco mono-isopropanolamide.

Further nonionic surfactants which can be included in shampoo compositions of the invention are the alkyl polyglycosides (APGs). Typically, APG is one which comprises an alkyl group connected (optionally via a bridging group) to a block of one or more glycosyl groups. Preferred APGs are defined by the following formula RO - (G)n wherein R is a branched or straight chain alkyl group which may be saturated or unsaturated and G is a saccharide group. R may represent a mean alkyl chain length of from about C5 to about C20. Preferably R represents a mean alkyl chain length of from about C8 to about C12. Most preferably the value of R lies between about 9.5 and about 10.5. G may be selected from C5 or C6 monosaccharide residues, and is preferably a glucoside. G may be selected from the group comprising glucose, xylose, lactose, fructose, mannose and derivatives thereof. Preferably G is glucose. The degree of polymerisation, n, may have a value of from about 1 to about 10 or more, preferably a value of from about 1.1 to about 2, most preferably a value of from about 1.3 to about 1.5. Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions of the invention include the C10-C18 N-alkyl (Cl-C6) polyhydroxy fatty acid amides, such as the C12-C18 N-methyl glucamides, as described for example in WO 92/06154 and US 5 194 639, and the N-alkoxy polyhydroxy fatty acid amides, such as C10-C18 N-(3-methoxypropyl) glucamide.

## 1.3 Amphoteric/zwitterionic Surfactant

Amphoteric or zwitterionic surfactant can be included in an amount ranging from 0.5 to about 8, preferably from 1 to 4 % w/w of the shampoo compositions of the invention.

Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine, lauryl betaine, cocamidopropyl betaine and sodium cocoamphoacetate.

A particularly preferred amphoteric or zwitterionic surfactant is cocamidopropyl betaine.

Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

## 1.4 Suspending agents

Preferably an aqueous shampoo composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked co-polymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. Carbopol 980 is the commonly used suspending agent though there is a growing desire to find an alternative. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

Suspending agent will generally be present in a shampoo composition of the invention at levels of from 0.1 to 10, preferably from 0.5 to 6, more preferably from 0.9 to 4 % w/w of the composition. Generally such suspending agents are present at around 2 % w/w of the composition.

**1.5 Water**

Shampoo compositions of the invention are generally aqueous, i.e. they have water or an aqueous solution or a lyotropic liquid crystalline phase as their major component. Suitably, the composition will comprise from 50 to 98, preferably from 60 to 90 % w/w of the composition.

Typically, shampoo compositions have a pH of around 5.5.

**1.6 Optional Ingredients**

The shampoo compositions of the invention might also contain the following optional ingredients: conditioning agents;

**1.6.1 Conditioning Agents**

Conditioning actives are often included in shampoo compositions. These are sometimes called '2-in-1' formulations. Conditioning actives fall into three classes:

- silicones (and cationic deposition polymers to assist in silicone deposition)
- cationic surfactants
- non-silicone oils

Where silicones are included, the composition is likely to also contain a cationic deposition polymer for enhancing deposition of the silicone. Further, a silicone-containing composition is likely to be lamellar as opposed to isotropic. Isotropic compositions do not deposit silicone effectively.

**1.6.1.1 Silicones**

The shampoo compositions of the invention can contain emulsified droplets of a silicone conditioning agent, for enhancing conditioning performance.

Suitable silicones include polydiorganosiloxanes, in particular polydimethylsiloxanes which have the CTFA designation dimethicone. Also suitable for use compositions of the invention (particularly shampoos and conditioners) are polydimethyl siloxanes having hydroxyl end groups, which have the CTFA designation dimethiconol. Also suitable for use in compositions of the invention are silicone gums having a slight degree of cross-linking, as are described for example in WO 96/31188.

Examples of suitable pre-formed emulsions include Xiameter MEM 1785 and microemulsion DC2-1865 available from Dow Corning. These are emulsions /microemulsions of dimethiconol. Cross-linked silicone gums are also available in a pre-emulsified form, which is advantageous for ease of formulation.

A further preferred class of silicones for inclusion in shampoos and conditioners of the invention are amino functional silicones. By "amino functional silicone" is meant a silicone containing at least one primary, secondary or tertiary amine group, or a quaternary ammonium group. Examples of suitable amino functional silicones include: polysiloxanes having the CTFA designation "amodimethicone".

Specific examples of amino functional silicones suitable for use in the invention are the aminosilicone oils DC2-8220, DC2-8166 and DC2-8566 (all ex Dow Corning). The most commonly used amino silicone is sourced from Dow Corning and is coded DC7134. Pre-formed emulsions of amino functional silicone are also available from suppliers of silicone oils such as Dow Corning and General Electric. Specific examples include DC939 Cationic Emulsion and the non-ionic emulsions DC2-7224, DC2-8467, DC2-8177 and DC2-8154 (all ex Dow Corning).

Suitable quaternary silicone polymers are described in EP-A-0 530 974. A preferred quaternary silicone polymer is K3474, ex Goldschmidt.

With some shampoos it is preferred to use a combination of amino and non amino functional silicones.

Emulsified silicones for use in the shampoo compositions of the invention will typically have an average silicone droplet size in the composition of less than 30, preferably less than 20, more preferably less than 10 micron, ideally from 0.01 to 1 micron. Silicone emulsions having an average silicone droplet size of about 0.15 micron are generally termed microemulsions.

Emulsified silicones for use in the conditioner compositions of the invention will typically have a size in the composition of less than 30, preferably less than 20, more preferably less than 15. Preferably the average silicone droplet is greater than 0.5 micron, more preferably greater than 1 micron, ideally from 2 to 8 micron.

Silicone particle size may be measured by means of a laser light scattering technique, for example using a 2600D Particle Sizer from Malvern Instruments.

The viscosity of the emulsified silicone itself (not the emulsion or the final hair conditioning composition) is typically at least 10,000, preferably at least 60,000, most preferably at least 500,000, ideally at least 1,000,000 cst at 25 °C. Preferably the viscosity does not exceed $10^9$ cst at 25 °C for ease of formulation.

The total amount of silicone is preferably from 0.01 to 10, more preferably from 0.1 to 5, most preferably 0.5 to 3 % w/w of the composition of the invention.

Also suitable are emulsions of amino functional silicone oils with non ionic and/or cationic surfactant.

Cationic deposition polymers are used to deposit the silicone droplets to the hair surface and hence enhance performance.

Suitable cationic polymers may be homopolymers which are cationically substituted or may be formed from two or more types of monomers. The weight average (Mw) molecular weight of the polymers will generally be between 100 000 and 2 million daltons. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerized in the amine form and then converted to ammonium by quaternization.

The cationic polymers can comprise mixtures of monomer units derived from amine-and/or quaternary ammonium-substituted monomer and/or compatible spacer monomers.

Suitable cationic polymers include, for example:

- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and co-polymers of unsaturated carboxylic acids having from 3 to 5 carbon atoms, (as described in US 4 009 256); and
- cationic polyacrylamides (as described in WO95/22311).

Other cationic polymers that can be used include cationic polysaccharide polymers, such as cationic cellulose derivatives, cationic starch derivatives, and cationic guar gum derivatives.

Cationic polysaccharide polymers suitable for use in compositions of the invention include monomers of the formula A-O-[R-N+(R1)(R2)(R3)X-] wherein A is an anhydroglucose residual group, such as a starch or cellulose anhydroglucose residual; R is an alkylene, oxyalkylene, polyoxyalkylene, or hydroxyalkylene group, or combination thereof; R1, R2 and R3 independently represent alkyl, aryl, alkylaryl, arylalkyl, alkoxyalkyl, or alkoxyaryl groups, each group containing up to about 18 carbon atoms; the total number of carbon atoms for each cationic moiety (i.e., the sum of carbon atoms in R1, R2 and R3) is preferably about 20 or less; and X is an anionic counterion.

Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the tradename Polymer LM-200.

Other suitable cationic polysaccharide polymers include quaternary nitrogen-containing cellulose ethers (e.g. as described in US 3 962 418), and copolymers of etherified cellulose and starch (e.g. as described in US 3 958 581).

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14, JAGUAR C15, JAGUAR C17 and JAGUAR C16 Jaguar CHT and JAGUAR C162.

Mixtures of any of the above cationic polymers may be used.

Cationic polymer will generally be present in a shampoo composition of the invention at levels of from 0.01 to 5, preferably from 0.05 to 1, more preferably from 0.08 to 0.5 % w/w of the weight of the compositions of the invention.

### 1.6.1.2 Cationic Surfactants

Cationic surfactants may be used in 2-in-1 shampoos to provide a conditioning benefit. However, since a shampoo composition is likely to also comprise anionic cleansing surfactants, the use of cationic surfactants is limited to compositions where the cationic surfactant is separated from the anionic phase by way of a stable conditioning gel phase made separately from the rest of the formulation and then incorporated afterwards.

### 1.6.1.3 Non-silicone Oils

These are typically hydrocarbon oils or fatty alcohols. A fatty alcohol is nearly always included in a conditioning composition and often included in 2-in-1 shampoos. Cetearyl alcohol is one of the preferred examples.

### 1.6.2 Fibre Actives

Fibre actives are provided to repair or coat the hair fibres. Examples are trehalose (a disaccharide), adipic acid (dicarboxylic acid) and gluconolactone.

### 1.6.3 Anti-dandruff Actives

There are two classes of anti-dandruff active: the azoles and the pyrithiones, both are active against the target fungi malasezzia spp. The azoles include ketoconazole and climbazole which are fat soluble actives. The pyrithiones include zinc pyrithione (ZPT) which is insoluble and delivered as a particle to the scalp.

Preferably, the antidandruff active is present at from 0.01 to 5, more preferably from 0.1 to 2.5 % w/w of the composition of the invention.

### 2.0 Hair Conditioning Compositions

The compositions of the invention may also be hair conditioning compositions (also known as conditioners). A conditioner which is to be used after a shampoo is known as a 'system conditioner' whereas one which is included in a shampoo composition is known as a '2-in-1'. Hair conditioning compositions may also be left on the head, i.e. not rinsed off after application. These are known as Leave-on-Treatments (LOTs) as opposed to Rinse-off-Treatments (ROTs).

The main ingredients in a system conditioner are the conditioning actives described above, the main actives being a cationic surfactant (e.g. behenyltrimmonium chloride), a silicone conditioning agent (e.g. aminosilicone (DC 7134)) and a non-silicone oil, usually a fatty alcohol (e.g. cetearyl alcohol).

Anti-dandruff actives may also be included in hair conditioning compositions of the invention.

### 2.1 Cationic Surfactants

Preferably, the cationic surfactants have the formula N+R1 R2R3R4 wherein R1, R2, R3 and R4 are independently (C1 to C30) alkyl or benzyl. Preferably, one, two or three of R1, R2, R3 and R4 are independently (C4 to C30) alkyl and the other R1, R2, R3 and R4 group or groups are (C1-C6) alkyl or benzyl. More preferably, one or two of R1, R2, R3 and R4 are independently (C6 to C30) alkyl and the other R1, R2, R3 and R4 groups are (C1-C6) alkyl or benzyl groups. Optionally, the alkyl groups may comprise one or more ester (-OCO- or -COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (e.g. oleyl). Alkyl groups are optionally ethoxylated on the alkyl chain with one or more ethyleneoxy groups.

Suitable cationic surfactants for use in conditioner compositions according to the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as GENAMIN CTAC, ex Hoechst Celanese. Another particularly useful cationic surfactant for use in conditioners according to the invention is behenyltrimethylammonium chloride, available commercially, for example as GENAMIN KDMP, ex Clariant.

Another example of a class of suitable cationic surfactants for use in the invention, either alone or together with one or more other cationic surfactants, is a combination of (i) and (ii) below:

(i) an amidoamine corresponding to the general formula (I) $R1CONH(CH_2)mN(R2)R3$
in which R1 is a hydrocarbyl chain having 10 or more carbon atoms, R2 and R3 are independently selected from hydrocarbyl chains of from 1 to 10 carbon atoms, and m is an integer from 1 to about 10; and
(ii) an acid.

As used herein, the term hydrocarbyl chain means an alkyl or alkenyl chain. Preferred amidoamine compounds are those corresponding to formula (I) in which R1 is a hydrocarbyl residue having from about 11 to about 24 carbon atoms, R2 and R3 are each independently hydrocarbyl residues, preferably alkyl groups, having from 1 to about 4 carbon atoms, and m is an integer from 1 to about 4. Preferably R2 and R3 are methyl or ethyl groups. Preferably m is 2 or 3, i.e. an ethylene or propylene group.

Preferred amidoamines useful herein include stearamido-propyldimethylamine, stearamidopropyldiethylamine, stearamidoethyldiethylamine, stearamidoethyldimethylamine, palmitamidopropyldimethylamine, palmitamidopropyl-diethylamine, palmitamidoethyldiethylamine, palmitamidoethyldimethylamine, behenamidopropyldimethyl-amine, behenamidopropyldiethylmine, behenamidoethyldiethyl-amine, behenamidoethyldimethylamine, arachidamidopropyl-dimethylamine, arachidamidopropyldiethylamine, arachid-amidoethyldiethylamine, arachidamidoethyldimethylamine, and mixtures thereof. Particularly preferred amidoamines useful herein are stearamidopropyldimethylamine, stearamidoethyldiethylamine, and mixtures thereof.

Commercially available amidoamines useful herein include: stearamidopropyldimethylamine with tradenames LEXAMINE S-13 available from Inolex (Philadelphia Pennsylvania, USA) and AMIDOAMINE MSP available from Nikko (Tokyo, Japan), stearamidoethyldiethylamine with a tradename AMIDOAMINE S available from Nikko, behenamidopropyldimethylamine with a tradename INCROMINE BB available from Croda (North Humberside, England), and various amidoamines with tradenames SCHERCODINE series available from Scher (Clifton New Jersey, USA).

The acid may be any organic or mineral acid which is capable of protonating the amidoamine in the conditioner composition. Suitable acids useful herein include hydrochloric acid, acetic acid, tartaric acid, fumaric acid, lactic acid, malic acid, succinic acid, and mixtures thereof. Preferably, the acid is selected from the group consisting of acetic acid, tartaric acid, hydrochloric acid, fumaric acid, lactic acid and mixtures thereof.

The primary role of the acid is to protonate the amidoamine in the hair treatment composition thus forming a tertiary amine salt (TAS) in-situ in the hair treatment composition. The TAS in effect is a non-permanent quaternary ammonium or pseudo-quaternary ammonium cationic surfactant. Suitably, the acid is included in a sufficient amount to protonate more than 95 mole % (20 °C) of the amidoamine present.

In conditioners of the invention, the level of cationic surfactant will generally range from 0.01 to 10, more preferably 0.05 to 7.5, most preferably 0.1 to 5 % by weight of the composition.

## 2.2 Silicone Conditioning Agent

The compositions of the invention can contain emulsified droplets of a silicone conditioning agent, for enhancing conditioning performance as previously described.

### 2.3 Non-silicone Oils

Compositions according to the present invention may also comprise a dispersed, non-volatile, water-insoluble, non-silicone oily conditioning agent. Preferably such non-silicone oily conditioning agents are present in the hair conditioning compositions of the invention. By "insoluble" is meant that the material is not soluble in water (distilled or equivalent) at a concentration of 0.1 % w/w at 25°C. Suitable non-silicone oils are selected from hydrocarbon oils, fatty esters and mixtures thereof.

Straight chain hydrocarbon oils will preferably contain from about 12 to about 30 carbon atoms. Also suitable are polymeric hydrocarbons of alkenyl monomers, such as C2-C6 alkenyl monomers. Specific examples of suitable hydrocarbon oils include paraffin oil, mineral oil, saturated and unsaturated dodecane, saturated and unsaturated tridecane, saturated and unsaturated tetradecane, saturated and unsaturated pentadecane, saturated and unsaturated hexadecane, and mixtures thereof. Branched-chain isomers of these compounds, as well as of higher chain length hydrocarbons, can also be used.

Suitable fatty esters are characterised by having at least 10 carbon atoms, and include esters with hydrocarbyl chains derived from fatty acids or alcohols, Monocarboxylic acid esters include esters of alcohols and/or acids of the formula R'COOR in which R' and R independently denote alkyl or alkenyl radicals and the sum of carbon atoms in R' and R is at least 10, preferably at least 20. Di- and trialkyl and alkenyl esters of carboxylic acids can also be used. Particularly preferred fatty esters are mono-, di- and triglycerides, more specifically the mono-, di-, and tri-esters of glycerol and long chain carboxylic acids such as C1-C22 carboxylic acids. Preferred materials include cocoa butter, palm stearin, sunflower oil, soyabean oil and coconut oil.

The oily or fatty material is suitably present at a level of from 0.05 to 10, preferably from 0.2 to 5, more preferably from about 0.5 to 3 % w/w of the composition of the invention.

### 2.4 Fatty Alcohols

Hair conditioning compositions of the invention will typically also incorporate a fatty alcohol. The combined use of fatty alcohols and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol in conditioners of the invention will generally range from 0.01 to 10, preferably from 0.1 to 8, more preferably from 0.2 to 7, most preferably from 0.3 to 6 % w/w by weight of the composition. The weight ratio of cationic surfactant to fatty alcohol is suitably from 1:1 to 1:10, preferably from 1:1.5 to 1:8, optimally from 1:2 to 1:5. If the weight ratio of cationic surfactant to fatty alcohol is too high, this can lead to eye irritancy from the composition. If it is too low, it can make the hair feel squeaky for some consumers.

### 3.0 Examples

Differential Scanning Calorimetry (DSC) and fibre mechanical measurements (dry tensile modulus, wet tensile modulus, wet break strength) were selected to evaluate damage repair and prevention after treatment with the compositions of the invention.

DSC measures the denaturation temperature of the $\alpha$-helical proteins in the microfibrils of the hair fibre. This temperature is influenced by the cross-link density of the surrounding matrix proteins (Wortmann et al., J. Cosmet. Sci., 53, 219-228 (2002)). A higher denaturation peak temperature indicates greater integrity of the fibre.

Bleaching would be expected to lead to a reduction in the wet modulus and wet break strength, and either a small decrease, negligible change or most commonly with moderate numbers of bleaching cycles, a small increase in the dry modulus. Repair of damage of the hair fibre would be expected to reverse these changes.

### Example 1

### Materials

Human natural white hair switches (International Hair Importers, USA)
Platine precision lightening powder (50 % persulphates, 24.1 % silicates and 2.6 %

ammonium chloride) (L'Oreal, France)
Excel cream peroxide, 9 %. (Excel (GS) Ltd., UK)
Sodium citrate (Sigma, UK)
Hydrogen peroxide, 3 % aq. (Sigma, UK)
(+)-catechin (Sigma, UK)
Dimethyl sulphoxide (DMSO) (Sigma, UK)
Horseradish peroxidase, 53 U/mg (1 Unit = 1 mg purpurogallin in 20 s at 20°C and pH 6) (HRP I, Sigma, UK)

**Methods**

**Hair bleaching**

Selected 2" hair switches were bleached twice or 8 times using L'Oreal Platine Precision lightening powder and Excel cream peroxide according to the manufacturers' instructions (30 minutes each treatment).

**Hair treatment**

300 μL sodium citrate buffer (100 mM pH 5.5, final concentration 60 mM)
50 μl hydrogen peroxide, 3%
50 μL (+)-catechin, 100mg/mL stock in DMSO

The reagents listed above were combined in a plastic trough. A hair switch was then placed in the trough and the solution was thoroughly massaged into the switch. The switch/reagents were then incubated at room temperature for 10 minutes. 100 μL horseradish peroxidase (HRP) (1 mg/mL in citrate buffer) was then added, rubbed into the switch and incubated at room temperature for 10 minutes. The switch was then rinsed twice with distilled water (30 s each rinse) and allowed to air dry. The treatment was repeated twice more (3 treatments in total).

**DSC**

Damage repair was assessed using DSC. A Mettler Toledo DSC1 analyser was used. The hair was shaved, using clippers, into 1-2 mm lengths. Approximately 5 mg of sample was weighed into a pressure resistant (20 bar), stainless steel, large volume pan (120 μL capacity). 50 μL of water was added and the pan was sealed. The samples were then mixed using a rotary mixer and left overnight to allow the water to equilibrate throughout the sample. Samples were run through a temperature programme of 100 - 180 °C at a rate of 5 °C/ min. The helix transition temperature was measured. Each sample was measured three times.

**Results**

The results are presented in table 1 and figure 1. All differences were significant (p =< 0.05) except * unbleached treated vs bleached treated x 2 (Student's t-test at 95 % confidence limits).

Table 1: Denaturation peak temperature (°C) for (+)-catechin, horseradish peroxidase, and hydrogen peroxide combination on natural white hair unbleached, and bleached x2 and x8. Standard errors at 95 % confidence limits are provided.

|  | **Unbleached** (°C) | **Bleached x2** (°C) | **Bleached x8** (°C) |
|---|---|---|---|
| No treatment | 146.77 +/- 0.14 | 137.80 +/- 0.28 | 130.38 +/- 0.67 |
| Treatment | 148.00 +/- 0.54 | 147.78 +/- 0.27 | 144.54 +/- 0.14 |

Analysis by DSC showed an increase in denaturation temperature for unbleached and bleached hair after treatment with the (+)-catechin, horseradish peroxidase, and hydrogen peroxide combination, indicating damage repair.

**Example 2**

**Materials (additional)**

0.5 M Britton-Robinson (BR) buffer

**Methods**

**Hair bleaching**

Selected 5" hair switches were bleached twice using L'Oreal Platine Precision lightening powder and Excel cream peroxide according to the manufacturers' instructions (30 minutes each treatment).

**Hair treatment**

1600 µL Britton-Robinson buffer (62.5 mM pH 6; final concentration 50 mM)
200 µL (+)-catechin (200 mg/mL stock in DMSO)
200 µL milliQ water

The reagents listed above were combined in a plastic universal pot. Each hair switch was wetted with milliQ water and placed into the pot, wetting the hair thoroughly. The pot was purged with nitrogen (to prevent oxidation) and incubated at 37 °C overnight (18 hours).

The next morning the switch was washed by swirling in a beaker of MilliQ water for approximately 2 minutes. The (+)-catechin /buffer mix was discarded. 1400 µL BR buffer, 200 µL milliQ water and 200 µL 3 % hydrogen peroxide were then placed into a clean universal pot and rubbed into the switch. The hair switch was placed into the pot (ensuring it was completely covered with solution) and incubated at 37 °C for 5 minutes.

200 µL HRP (1 mg/mL in BR buffer, 62.5 mM pH 6) was then added and the combined solution was rubbed into the switch. The hair switch was placed into the pot and incubated at 37 °C for 5 minutes. The switch was then washed by swirling in a beaker of MilliQ water for approximately 2 minutes. After washing the hair was dried with a hairdryer and combed.

The above method was repeated four times (5 treatments in total). The hair was shampooed (30 s shampoo, 60 s rinse) between each treatment step.

**Fibre mechanical measurements**

Damage repair was assessed using fibre mechanical measurements. For each hair fibre the following measurements were taken: (a) dry tensile modulus (Young's modulus at 2 % extension at 20 °C and 50 % relative humidity); (b) wet tensile modulus (Young's modulus at 2 % extension immersed in water); and (c) wet breakage (100 % extension immersed in water). Hairs were equilibrated for at least 2 hours before measurements were taken. 50 fibres per sample were tested.

**Results**

The results are presented in table 2 and figures 2a and 2b. In figures 2a and 2b, (* $p = < 0.01$ and ** $p = < 0.001$ (Student's t-test at 95 % confidence limits).

Table 2: Fibre mechanical measurements on for (+)-catechin, horseradish peroxidase, and hydrogen peroxide combination on unbleached and bleached x2 natural white hair. Dry and wet tensile moduli, and wet break strength test ($N/m^2$). Standard errors at 95 % confidence limits are provided.

| | Unbleached hair | | Bleached x2 hair | |
|---|---|---|---|---|
| | No treatment | Treatment | No treatment | Treatment |
| Dry tensile modulus ($N/m^2$) | 4.86E+09 +/- 3.53E+08 | 5.19E+09 +/- 3.76E+08 | 4.87E+09 +/- 3.15E+08 | 5.61E+09 +/- 3.38E+08 |

(continued)

| | Unbleached hair | | Bleached x2 hair | |
|---|---|---|---|---|
| | No treatment | Treatment | No treatment | Treatment |
| Wet tensile modulus (N/m$^2$) | 2.25E+09 +/- 2.06E+08 | 2.41E+09 +/- 2.48E+08 | 1.76E+09 +/- 2.26E+08 | 2.04E+09 +/- 2.74E+08 |
| Wet break strength (N/m$^2$) | 1.71E+09 +/- 1.75E+08 | 1.81E+09 +/- 1.90E+08 | 1.34E+09 +/- 1.75E+08 | 1.61E+09 +/- 1.82E+08 |

Analysis of mechanical properties of unbleached and double bleached natural white hair before and after treatment with the (+)-catechin, horseradish peroxidase, and hydrogen peroxide combination shows (i) an increase in dry tensile modulus after treatment indicating increased fibre stiffness, (ii) an increase in wet tensile modulus after treatment indicating damage repair, and (iii) an increase in wet break strength after treatment indicating damage repair.

**Example 3**

**Materials (additional)**

Soy bean peroxidase, 1840 U/mg (1 Unit = 1 $\mu$M change per minute at 30 °C and pH 6 guaiacol (Bio-Research Products, USA)
Laccase 51003 (*Myceliophthora thermophila*), 1000 U (LAMU)/g (ml) (1 LAMU = 1 $\mu$M change per minute at 30 °C and pH 7.5 syringaldazine (Novozymes, Denmark)

**Methods**

**Hair bleaching**

Selected 2" hair switches were bleached once using L'Oreal Platine Precision lightening powder and Excel cream peroxide according to the manufacturers' instructions (30 minutes each treatment).

**Hair treatment**

4200 $\mu$L Britton-Robinson buffer (62.5 mM pH 5; final concentration 50 mM)
600 $\mu$l hydrogen peroxide 3% (soybean peroxidase-treated sample and catechin / H2O2 -treated sample) or 600 $\mu$L milliQ water (laccase-treated sample)
600 $\mu$L (+)-catechin, 200mg/mL stock in DMSO

The reagents listed above were combined in a 15 mL Falcon centrifuge tube. The hair switch was placed into the tube and squashed down, wetting the hair thoroughly. The switch/reagents were then incubated at 32 °C for 15 minutes. 30 units soy bean peroxidase (SBP) or laccase (in Britton-Robinson buffer pH 5, total volume 600 $\mu$L) were then added and rubbed into the switch. The hair switch was placed back into the tube and incubated at 32 °C for another 15 minutes. After incubation the switch was washed by swirling in a beaker of milliQ water for approx. 2 minutes. The hair was then dried with a hairdryer and combed. A second experiment was carried out as above substituting the enzyme with 600 $\mu$L BR buffer pH 5. All other steps were as set forth above.

**DSC**

Damage repair was assessed by DSC using the method described above (see example 1). Control samples (no treatment) and (+)-catechin only (but still with hydrogen peroxide) treated samples were measured three times. (+)-Catechin / enzyme-treated samples were measured 8 times.

## Results

The results are summarised in table 3 and figures 3a and 3b. Figure 3a shows the denaturation peak temperature (°C) for the (+)-catechin and hydrogen peroxide combination (3a) on natural white hair unbleached, and bleached x1, whilst figure 3b shows the same values for the ((+)- catechin, soy bean peroxidase, and hydrogen peroxide combination, and the (+)-catechin and laccase combination on natural white hair unbleached, and bleached x1. With standard errors at 95 % confidence limits in figure 3a ** untreated control $p < 0.0001$, and figure 3b (* (+)-catechin /laccase $p = 0.0029$, and ** (+)-catechin / laccase $p = 0.0072$.

Table 3: Denaturation peak temperature (°C) for (+)-catechin, soy bean peroxidase, and hydrogen peroxide combination; (+)- catechin and laccase combination; and (+)- catechin and hydrogen peroxide combination; on natural white hair unbleached, and bleached x1. Standard errors at 95 % confidence limits are provided.

|  | Unbleached (°C) | Bleached x1 (°C) |
| --- | --- | --- |
| No treatment | 146.46 +/- 0.05 | 142.09 +/- 0.34 |
| (+)- Catechin / $H_2O_2$ | 149.27 +/- 0.10 | 150.62 +/- 0.10 |
| (+)- Catechin / $H_2O_2$ / soy bean peroxidase | 148.83 +/- 0.30 | 150.58 +/- 0.34 |
| (+)- Catechin / laccase | 148.36 +/- 0.18 | 149.89 +/- 0.47 |

Soy bean peroxidase in combination with $H_2O_2$ is more effective than laccase for repairing hair damage when a flavonoid, e.g. (+)-catechin, is used as a substrate. For unbleached and bleached hair, the DSC denaturation temperature for hair treated with catechin, soy bean peroxidase and $H_2O_2$ is significantly greater than the DSC denaturation temperature for hair treated with (+)-catechin and laccase. Analysis by DSC shows an increase in denaturation temperature for unbleached and bleached hair after treatment with (+)-catechin (with $H_2O_2$ but no enzyme), indicating strengthening of the fibre.

## Example 4

### Materials (additional)

Human dark brown hair switches (International Hair Importers, USA)
Horseradish peroxidase, 274 U/mg (1 Unit = 1 mg purpurogallin in 20 s at 20°C and pH 6) (HRP VI, Sigma, UK)

### Methods

### Hair bleaching

Selected 2" hair switches were bleached once using L'Oreal Platine Precision lightening powder and Excel cream peroxide according to the manufacturers' instructions (30 minutes each treatment).

### Hair treatment

4200 μL Britton-Robinson buffer (62.5 mM pH 5; final concentration 50 mM)
600 μl hydrogen peroxide 3% (SBP- or HRP-treated sample and (+)-catechin /$H_2O_2$ treated sample) or 600 μL milliQ water (laccase-treated sample and (+)-catechin treated sample)
600 μL (+)-catechin, 100mg/mL stock in DMSO

The reagents listed above were combined in 15 mL Falcon centrifuge tubes. Unbleached and bleached hair switches were placed into the tubes and squashed down, wetting the hair thoroughly (one switch per tube). The switch/reagents were then incubated at 32 °C for 15 minutes. 30 Units horseradish peroxidase (HRP), soy bean peroxidase (SBP) or laccase (in Britton-Robinson buffer pH 5, total volume 600 □L) were then added as appropriate to the tubes. 600 □L Britton-Robinson buffer pH 5 were added to the tubes containing the (+)-catechin and (+)-catechin / $H_2O_2$ treated

switches. The hair switches were then incubated at 32 °C for another 15 minutes. After incubation the switches were washed by swirling in a beaker of milliQ water for approximately 1 minute. The hair was then shampoo washed by rubbing with shampoo for 30 seconds and subsequently rinsing under tap water for 60 seconds. The hair was then dried with a hairdryer and combed. The above method was repeated 4 times (5 treatments in total). Untreated switches were washed 5 times with shampoo, rinsed and dried as described above.

**DSC**

Denaturation temperature was assessed by DSC using the method described in example 1. Samples were measured three times.

**Results**

The results are summarised in table 4 and figures 4a and 4b. Figure 4a shows denaturation peak temperature (°C) for (+)-catechin; (+)-catechin and hydrogen peroxide combination; (+)-catechin, horseradish peroxidase, and hydrogen peroxide combination; and the (+)-catechin and laccase combination on dark brown hair unbleached. (** untreated control $p < 0.01$ for standard error at 95 % confidence limits). Figure 4b shows denaturation peak temperature (°C) for (+)-catechin; (+)-catechin and hydrogen peroxide combination; (+)-catechin, horseradish peroxidase, and hydrogen peroxide combination; (+)-catechin, soy bean peroxidase, and hydrogen peroxide combination; and the (+)-catechin and laccase combination on dark brown hair bleached x1 (** untreated control $p < 0.0001$ for standard error at 95 % confidence limits). Student's T-tests were performed on all the data in table 4 giving the following results for unbleached hair:

HRP vs laccase      0.0012
control vs HRP      less than 0.01
control vs H2O2     less than 0.01

control vs laccase less than 0.01 and for bleached hair:

HRP vs SBP         0.12
HRP vs laccase     0.043
SBP vs laccase     0.05
control vs HRP     less than 0.0001
control vs SBP     less than 0.0001
control vs laccase less than 0.0001

Table 4: Denaturation peak temperature (°C) for (+)-catechin, horseradish peroxidase, and hydrogen peroxide combination; (+)-catechin, soy bean peroxidase, and hydrogen peroxide combination; (+)-catechin and laccase combination; (+)-catechin and hydrogen peroxide combination: and (+)-catechin alone; on dark brown hair unbleached, and bleached x1. Standard errors at 95 % confidence limits are provided.

|  | **Unbleached** (°C) | **Bleached** x1 (°C) |
|---|---|---|
| No treatment | 147.38 +/- 0.72 | 143.60 +/- 0.65 |
| (+)-Catechin | 151.16 +/- 0.49 | 153.38 +/- 0.43 |
| (+)-Catechin / H2O2 | 152.06 +/- 0.18 | 154.18 +/- 0.15 |
| (+)-Catechin / H2O2 /HRP | 151.96 +/- 0.09 | 154.76 +/- 0.41 |
| (+)-Catechin / H2O2 / SBP | n/a | 154.19 +/- 0.06 |
| (+)-Catechin / laccase | 150.80 +/- 0.18 | 153.85 +/- 0.16 |
| n/a = not available | | |

Analysis by DSC shows treatment with peroxidase/hydrogen peroxide combinations led to a greater increase in denaturation temperature than a treatment with laccase for bleached hair. This was also observed for HRP for unbleached hair. Treatment with any of the tested enzymes was observed to lead to significant increases in the denaturation temperature.

### Example 5

### Materials (additional)

> Myricetin (Sigma, UK)
> Gossypetin (Extrasynthese, France)
> Luteolin (Sigma, UK)
> Apigenin (Sigma, UK)
> Naringenin (Sigma, UK)
> Horseradish peroxidase, 274 U/mg (1 Unit = 1 mg purpurogallin in 20 s at 20°C and pH 6) (HRP VI, Sigma, UK)

### Methods

### Hair bleaching

Selected 2" hair switches were bleached twice using L'Oreal Platine Precision lightening powder and Excel cream peroxide according to the manufacturers' instructions (30 minutes each treatment).

### Hair treatment

> 4200 $\mu$L Britton-Robinson buffer (62.5 mM pH 5; final concentration 50 mM)
> 600 $\mu$l hydrogen peroxide 3% (HRP-treated samples) or 600 $\mu$L milliQ water (laccase-treated samples)
> 600 $\mu$L flavanoid ((+)-catechin, myricetin, gossypetin, luteolin, apigenin or naringenin), 10mg/mL stock in DMSO

The reagents listed above were combined in 15 mL Falcon centrifuge tubes. Bleached hair switches were placed into the tubes and squashed down, wetting the hair thoroughly (one switch per tube). The switch/reagents were then incubated at 32 °C for 15 minutes. 30 Units horseradish peroxidase (HRP) or laccase (in Britton-Robinson buffer pH 5, total volume 600 □L) were then added as appropriate to the tubes. The hair switches were then incubated at 32 °C for another 15 minutes. After incubation the switches were washed by rinsing under running tap water for 1 minute. The hair was then shampoo washed by rubbing with shampoo for 30 seconds and subsequently rinsing under tap water for 60 seconds. The hair was then dried with a hairdryer and combed. The above method was repeated twice (3 treatments in total). Untreated switches were washed 3 times with shampoo, rinsed and dried as described above.

### DSC

Denaturation temperature was assessed by DSC using the method described in example 1. Untreated samples were measured three times. All other samples were measured six times.

### Results

The results are summarised in tables 5a, 5b and 5c and figures 5a and 5b. Figure 5a shows the denaturation peak temperature (°C) for (+)-catechin and laccase combination; myricetin, hydrogen peroxide and horseradish peroxidase combination; and gossypetin, hydrogen peroxide and horseradish peroxidase combination on double bleached natural white hair. (** versus untreated control $p < 0.0001$ for standard error at 95 % confidence limits). Figure 5b shows the denaturation peak temperature (°C) for (+)-catechin and laccase combination; and luteolin, hydrogen peroxide and horseradish peroxidase combination on double bleached natural white hair (** versus untreated control $p < 0.0001$ for standard error at 95 % confidence limits). Student's T-tests were performed on all the data in tables 5a, 5b and 5c giving the following results for bleached hair:

| | |
|---|---|
| myricetin / H2O2 / HRP vs catechin / laccase | 0.025 |
| gossypetin / H2O2 / HRP vs catechin / laccase | 0.0082 |
| control vs catechin / laccase | less than 0.0001 |
| control vs myricetin / H2O2 / HRP | less than 0.0001 |

(continued)

| | |
|---|---|
| control vs gossypetin / H2O2 / HRP | less than 0.0001 |
| luteolin / H2O2 / HRP vs catechin / laccase | 0.027 |
| apigenin / H2O2 / HRP vs catechin / laccase | 0.068 |
| control vs catechin / laccase | less than 0.0001 |
| control vs luteolin / H2O2 / HRP | less than 0.0001 |
| control vs apigenin / H2O2 / HRP | less than 0.0001 |
| naringenin / H2O2 / HRP vs catechin / laccase | 0.87 |
| control vs catechin / laccase | less than 0.0001 |
| control vs naringenin / H2O2 / HRP | less than 0.0001 |

Table 5a: Denaturation peak temperature (°C) for (+)-catechin and laccase combination; myricetin, hydrogen peroxide and horseradish peroxidase combination; and gossypetin, hydrogen peroxide and horseradish peroxidase combination on double bleached natural white hair. Standard errors at 95 % confidence limits are provided.

| | Bleached x2 (°C) |
|---|---|
| No treatment | 136.97 +/- 0.04 |
| (+)-Catechin / laccase | 148.85 +/- 0.43 |
| Myricetin / $H_2O_2$ / HRP | 149.75 +/- 0.64 |
| Gossypetin / $H_2O_2$ / HRP | 149.61 +/- 0.29 |

Table 5b: Denaturation peak temperature (°C) for (+)-catechin and laccase combination; luteolin, hydrogen peroxide and horseradish peroxidase combination; and apigenin, hydrogen peroxide and horseradish peroxidase combination on double bleached natural white hair. Standard errors at 95 % confidence limits are provided

| | Bleached x2 (°C) |
|---|---|
| No treatment | 136.97 +/- 0.04 |
| (+)-Catechin / laccase | 148.00 +/- 0.66 |
| Luteolin / $H_2O2$ / HRP | 148.88 +/- 0.38 |
| Apigenin / $H_2O_2$ / HRP | 148.94 +/- 0.79 |

Table 5c: Denaturation peak temperature (°C) for (+)-catechin and laccase combination; and naringenin, hydrogen peroxide and horseradish peroxidase combination; on double bleached natural white hair. Standard errors at 95 % confidence limits are provided

| | Bleached x2 (°C) |
|---|---|
| No treatment | 136.97 +/- 0.04 |
| (+)-Catechin / laccase | 149.42 +/- 0.61 |
| Naringenin / $H_2O_2$ / HRP | 149.47 +/- 0.32 |

Analysis by DSC shows that treatment with myricetin, hydrogen peroxide and horseradish peroxidase combination; gossypetin, hydrogen peroxide and horseradish peroxidase combination; and luteolin, hydrogen peroxide and horseradish peroxidase combination led to a greater increase in denaturation temperature than treatment with (+)-catechin / laccase for bleached hair. The myricetin, gossypetin and luteolin combinations colour hair (see Example 6). Treatment with apigenin, hydrogen peroxide and horseradish peroxidase combination; and naringenin, hydrogen peroxide and horseradish peroxidase combination did not lead to a greater increase in denaturation temperature than treatment with (+)-catechin / laccase for bleached hair. The apigenin and naringenin combinations do not colour hair (see Example 6). Treatment with both enzymes was observed to lead to significant increases in the denaturation temperature.

**Example 6**

**Materials (additional)**

No additional materials.

**Methods**

**Hair bleaching**

Selected 2" hair switches were bleached twice using L'Oreal Platine Precision lightening powder and Excel cream peroxide according to the manufacturers' instructions (30 minutes each treatment).

**Hair treatment**

4200 µL Britton-Robinson buffer (62.5 mM pH 5; final concentration 50 mM)
600 µL hydrogen peroxide 3% (HRP-treated samples) or 600 µL milliQ water (laccase-treated samples)
600 µL flavonoid ((+)-catechin, myricetin, gossypetin, luteolin, apigenin or naringenin), 10mg/mL stock in DMSO

Prior to treatment, the colour (L*a*b*) of each hair switch was recorded using a Minolta CM-2600d spectrophotometer. The reagents listed above were then combined in 15 mL Falcon centrifuge tubes. The hair switches were placed into the tubes and squashed down, wetting the hair thoroughly (one switch per tube). The switch/reagents were then incubated at 32 °C for 15 minutes. 30 Units horseradish peroxidase (HRP) or laccase (in Britton-Robinson buffer pH 5, total volume 600 µL) were then added as appropriate to the tubes. The hair switches were then incubated at 32 °C for another 15 minutes. After incubation the switches were washed by rinsing under running tap water for 1 minute. The hair was then shampoo washed by rubbing with shampoo for 30 seconds and subsequently rinsing under tap water for 60 seconds. The hair was then dried with a hairdryer and combed. The above method was repeated twice (3 treatments in total). After each treatment the colour of each switch (L*a*b*) was recorded and ΔE was calculated using the following equation:

$$\Delta E \;=\; \sqrt{(L^{*}_{D} - L^{*}_{B})^{2} \;+\; (a^{*}_{D} - a^{*}_{B})^{2} \;+\; (b^{*}_{D} - b^{*}_{B})^{2}}$$

where B = blank (undyed)
D = dyed

Untreated switches were washed 3 times with shampoo, rinsed and dried as described above.

**Results**

The ΔE values for each switch after three treatments are shown in table 6. A ΔE value of 5 or above is considered to colour hair. Treatment with (+)- catechin / laccase, myricetin / H2O2 / HRP, gossypetin / H2O2 / HRP and luteolin / H2O2 / HRP coloured hair. Treatment with apigenin / H2O2 / HRP and naringenin / H2O2 / HRP did not colour hair.

Table 6: ΔE values for double bleached hair natural white hair treated three times with (+)-catechin and laccase combination; myricetin, hydrogen peroxide and horseradish peroxidase combination; gossypetin, hydrogen peroxide and horseradish peroxidase combination; luteolin, hydrogen peroxide and horseradish peroxidase combination; apigenin, hydrogen peroxide and horseradish peroxidase combination; and naringenin, hydrogen peroxide and horseradish peroxidase combination.

|  | ΔE |
|---|---|
| No treatment | 1.10 |
| (+)-Catechin / laccase | 21.40 |
| Myricetin / $H_2O_2$ / HRP | 31.46 |
| Gossypetin / $H_2O_2$ / HRP | 38.45 |

(continued)

| | $\Delta$E |
|---|---|
| Luteolin / / $H_2O_2$ / HRP | 13.08 |
| Apigenin / $H_2O_2$ / HRP | 1.72 |
| Naringenin / $H_2O_2$ / HRP | 2.44 |

**Claims**

1. A method of strengthening hair fibres, the method comprising the step of applying a hair composition comprising:

   (a) A flavonoid;
   (b) A hydrogen peroxide generator or hydrogen peroxide; and
   (c) A peroxidase;

   wherein the composition has a pH of 3 to 9, preferably 4.5 to 7, more preferably less than or equal to 6,
   wherein the $\Delta$E value of the hair fibres before and after application of the hair composition is at least +5.

2. Use of a hair composition comprising:

   (a) A flavonoid; and
   (b) A hydrogen peroxide generator or hydrogen peroxide; and
   (c) A peroxidase;

   wherein the composition has a pH of 3 to 9, preferably 4.5 to 7, more preferably less than or equal to 6,
   wherein the $\Delta$E value of the hair fibres before and after application of the hair composition is at least +5,
   for strengthening hair fibres.

3. A method or use according to claim 1 or claim 2, wherein the flavonoid is selected from the group consisting of flavones, isoflavones, flavans, isoflavans, flavanones, flavonols, flavan-3-ols, dihydroflavonol, flavanonols, anthocyanidins, proanthocyanidins, auron, chalcone, dihydrochalcone, flavonolignans, and derivatives thereof.

4. A method or use according to 3,
   wherein the flavone is selected from the group consisting of luteolin and chrysin;
   wherein the isoflavone is selected from the group consisting of daidzein, genistein and formononetin;
   wherein the flavan is 4'-hydroxy-5,6-dimethoxyflavan;
   wherein the isoflavan is glabridin or licoricidin;
   wherein the flavanone is eriodictyol;
   wherein the flavonol is selected from the group consisting of myricetin, kaempferol, gossypetin and quercetin;
   wherein the flavan-3-ol is selected from the group consisting of catechin, theaflavin, gallocatechin, catechin gallate, gallocatechin gallate, epicatechin, epigallocatechin, epigallocatechin gallate and epigallocatechin gallate; wherein the dihydroflavonol is taxifolin or aromadendrin;
   wherein the anthocyanidin is selected from the group consisting of cyanidin, delphinidin, pelargonidin and malvidin;
   wherein the aurone is sulphuretin;
   wherein the chalcone is 2'-hydroxy-4-methoxy-chalcone;
   wherein the dihydrochalcone is phloretin or phloridzin and
   wherein the flavonolignan is silibinin or silichristin.

5. A method according to claim 1 or claim 2, wherein the flavonoid is selected from the group consisting of silibinin, silandrin, 3,4-dihydroxyflavone, hesperidin (glycoside), naringin (naringenin glycoside), amentoflavone, rutin, eriodictyol-7-O-glucoside, quercitrin, kaempferol, pinostrobin and biochanin A.

6. A method according to claim 1 or claim 2, wherein the flavonoid is selected from the group consisting of (+)-catechin, myricetin, gossypetin and luteolin.

7. A method or use according to any one of the preceding claims, wherein the peroxidase is a non-animal haem peroxidase from class II (fungi) or class III (plants and algae).

8. A method or use according claim 7, wherein the peroxidase is obtained from the group consisting of Arabidopsis thaliana, horse radish, barley, peanut soybean, tobacco, and turnip (plants), Chlorophyta spirogyra (green algae), Arthromyces ramosus and Corprinus cinereus (fungi).

9. A method or use according to any one of the preceding claims, wherein the hair colour composition comprises 0.01 - 10, preferably 0.1 - 5 % w/w flavonoid.

10. A method or use according to any one of the preceding claims wherein the hair colour composition comprises 0.0001 - 3 preferably 0.001 - 1, most preferably 0.01 - 1 % w/w hydrogen peroxide.

11. A method or use according to any one of the preceding claims, wherein the hair colour composition comprises 0.0001 - 5, preferably 0.001 - 1 % w/w peroxidase.

12. A method or use according to any one of claims 1 to 10, wherein the hydrogen peroxide generator comprises a hydrogen peroxide generating oxidase, a substrate and oxygen.

13. A method or use according to any one of the preceding claims wherein the hair colour composition additionally comprises a metal ion suitable for coordinating to the flavonoid or oxidative product of the flavonoid, the metal ion preferably selected from the group consisting of iron (II), iron (III), copper (I), copper (II), copper (III) and aluminium (III).

14. A method or use according to any one of the preceding claims wherein the hair colour composition is a shampoo composition or hair conditioning composition.

**Patentansprüche**

1. Verfahren zur Stärkung von Haarfasern, wobei das Verfahren den Schritt des Auftragens einer Haarzusammensetzung umfasst, die

   (a) ein Flavonoid,
   (b) einen Entwickler von Wasserstoffperoxid oder Wasserstoffperoxid und
   (c) eine Peroxidase

umfasst,
wobei die Zusammensetzung einen pH von 3 bis 9, vorzugsweise von 4,5 bis 7, bevorzugter von weniger als oder gleich 6 aufweist,
wobei der $\Delta$E-Wert der Haarfasern vor und nach dem Auftragen der Haarzusammensetzung mindestens +5 beträgt.

2. Verwendung einer Haarzusammensetzung, die

   (a) ein Flavonoid und
   (b) einen Entwickler von Wasserstoffperoxid oder Wasserstoffperoxid und
   (c) eine Peroxidase

umfasst,
wobei die Zusammensetzung einen pH von 3 bis 9, vorzugsweise von 4,5 bis 7, bevorzugter von weniger als oder gleich 6 aufweist,
wobei der $\Delta$E-Wert der Haarfasern vor und nach dem Auftragen der Haarzusammensetzung mindestens +5 beträgt, zur Stärkung von Haarfasern.

3. Verfahren oder Verwendung nach Anspruch 1 oder 2, wobei das Flavonoid aus der Gruppe, die aus Flavonen, Isoflavonen, Flavanen, Isoflavanen, Flavanonen, Flavonolen, Flavan-3-olen, Dihydroflavonol, Flavanonolen, Anthocyanidinen, Proanthocyanidinen, Auron, Chalcon, Dihydrochalcon, Flavonolignanen und Derivaten davon besteht, ausgewählt ist.

4. Verfahren oder Verwendung nach Anspruch 3,

wobei das Flavon aus der aus Luteolin und Chrysin bestehenden Gruppe ausgewählt ist,

wobei das Isoflavon aus der aus Daidzein, Genistein und Formononetin bestehenden Gruppe ausgewählt ist,

wobei das Flavan 4'-Hydroxy-5,6-dimethoxyflavan ist,

wobei das Isoflavan Glabridin oder Licoricidin ist,

wobei das Flavanon Eriodictyol ist,

wobei das Flavonol aus der Gruppe, die aus Myricetin, Kaempferol, Gossypetin und Quercetin besteht, ausgewählt ist,

wobei das Flavan-3-ol unter der Gruppe, die aus Catechin, Theaflavin, Gallocatechin, Catechingallat, Gallocatechingallat, Epicatechin, Epigallocatechin, Epigallocatechingallat und Epigallocatechingallat besteht, ausgewählt ist,

wobei das Dihydroflavonol Taxifolin oder Aromadendrin ist,

wobei das Anthocyanidin aus der Gruppe, die aus Cyanidin, Delphinidin, Pelargonidin und Malvidin besteht, ausgewählt ist,

wobei das Auron Sulphuretin ist,

wobei das Chalcon 2'-Hydroxy-4-methoxychalcon ist,

wobei das Dihydrochalcon Phloretin oder Phloridzin ist und

wobei das Flavonolignan Silibinin oder Silichristin ist.

5. Verfahren nach Anspruch 1 oder Anspruch 2, wobei das Flavonoid aus der Gruppe, die aus Silibinin, Silandrin, 3,4-Dihydroxyflavon, Hesperidin (Glycosid), Naringin (Naringeninglycosid), Amentoflavon, Rutin, Eriodictyol-7-O-glucosid, Quercitrin, Kaempferol, Pinostrobin und Biochanin A besteht, ausgewählt ist.

6. Verfahren nach Anspruch 1 oder Anspruch 2, wobei das Flavonoid aus der Gruppe, die aus (+)-Catechin, Myricetin, Gossypetin und Luteolin besteht, ausgewählt ist.

7. Verfahren oder Verwendung nach irgendeinem der vorhergehenden Ansprüche, wobei die Peroxidase eine nicht-tierische Häm-Peroxidase der Klasse II (Pilz) oder Klasse III (Pflanzen und Algen) ist.

8. Verfahren oder Verwendung nach Anspruch 7, wobei die Peroxidase aus der Gruppe, die aus Arabidopsis thaliana, Meerrettich, Gerste, Erdnuss, Sojabohnen, Tabak und Rüben (Pflanzen), Chlorophyta spirogyra (grüne Algen), Arthromyces ramosus und Corprinus cinereus (Pilze) besteht, erhalten wird.

9. Verfahren oder Verwendung nach irgendeinem der vorhergehenden Ansprüche, wobei die Haarfärbezusammensetzung 0,01-10, vorzugsweise 0,1 bis 5% Gew./Gew. Flavonoid umfasst.

10. Verfahren oder Verwendung nach irgendeinem der vorhergehenden Ansprüche, wobei die Haarfärbezusammensetzung 0,0001 bis 3, vorzugsweise 0,001 bis 1, höchst bevorzugt 0,01 bis 1% Gew./Gew. Wasserstoffperoxid umfasst.

11. Verfahren oder Verwendung nach irgendeinem der vorhergehenden Ansprüche, wobei die Haarfärbezusammensetzung 0,0001 bis 5, vorzugsweise 0,001 bis 1% Gew./Gew. Peroxidase umfasst.

12. Verfahren oder Verwendung nach irgendeinem der Ansprüche 1 bis 10, wobei der Entwickler von Wasserstoffperoxid eine Wasserstoffperoxid entwickelnde Oxidase, ein Substrat und Sauerstoff umfasst.

13. Verfahren oder Verwendung nach irgendeinem der vorhergehenden Ansprüche, wobei die Haarfärbezusammensetzung zusätzlich ein Metallion umfasst, das zum Koordinieren an das Flavonoid oder das oxidative Produkt des Flavonoids geeignet ist, wobei das Metallion vorzugsweise aus der Gruppe, die aus Eisen (II), Eisen (III), Kupfer (I), Kupfer (II), Kupfer (III) und Aluminium (III) besteht, ausgewählt ist.

14. Verfahren oder Verwendung nach irgendeinem der vorhergehenden Ansprüche, wobei die Haarfärbezusammensetzung eine Shampoozusammensetzung oder eine Haarkonditionierzusammensetzung ist.

**Revendications**

1. Procédé de renforcement de fibres de cheveux, le procédé comprenant l'étape d'application d'une composition pour cheveux comprenant :

(a) un flavonoïde ;
(b) un générateur de peroxyde d'hydrogène ou du peroxyde d'hydrogène ; et
(c) une peroxydase ;

dans lequel la composition présente un pH de 3 à 9, de préférence de 4,5 à 7, encore mieux inférieur ou égal à 6, dans lequel la valeur ΔE des fibres de cheveux avant et après l'application de la composition pour cheveux est d'au moins +5.

2. Utilisation d'une composition pour cheveux comprenant :

(a) un flavonoïde ; et
(b) un générateur de peroxyde d'hydrogène ou du peroxyde d'hydrogène ; et
(c) une peroxydase ;

dans laquelle la composition présente un pH de 3 à 9, de préférence de 4,5 à 7, encore mieux inférieur ou égal à 6, dans laquelle la valeur ΔE des fibres de cheveux avant et après l'application de la composition pour cheveux est d'au moins +5,
pour renforcer des fibres de cheveux.

3. Procédé ou utilisation selon la revendication 1 ou la revendication 2, dans lequel le flavonoïde est choisi dans le groupe constitué de flavones, isoflavones, flavanes, isoflavanes, flavanones, flavonols, flavan-3-ols, dihydroflavonol, flavanonols, anthocyanidines, proanthocyanidines, aurone, chalcone, dihydrochalcone, flavonolignanes, et dérivés de ceux-ci.

4. Procédé ou utilisation selon la revendication 3,
dans lequel la flavone est choisie dans le groupe constitué de lutéoline et chrysine ;
dans lequel l'isoflavone est choisie dans le groupe constitué de daidzéine, génistéine et formononétine ;
dans lequel le flavane est le 4'-hydroxy-5,6-diméthoxyflavane ; dans lequel l'isoflavane est la glabridine ou la licoricidine ;
dans lequel la flavonone est l'ériodictyol ;
dans lequel le flavonol est choisi dans le groupe constitué de myricétine, kaempférol, gossypétine et quercétine ;
dans lequel le flavan-3-ol est choisi dans le groupe constitué de catéchine, théaflavine, gallocatéchine, gallate de catéchine, gallate de gallocatéchine, épicatéchine, épigallocatéchine, gallate d'épigallocatéchine et gallate d'épigallocatéchine ;
dans lequel le dihydroflavonol est la taxifoline ou l'aromadendrine ;
dans lequel l'anthocyanidine est choisie dans le groupe constitué de cyanidine, delphinidine, pelargonidine et malvidine ;
dans lequel l'aurone est la sulfurétine ;
dans lequel la chalcone est la 2'-hydroxy-4-méthoxy-chalcone ; dans lequel la dihydrochalcone est la phlorétine ou la phloridzine et
dans lequel le flavonolignane est la silibinine ou la silichristine.

5. Procédé selon la revendication 1 ou la revendication 2, dans lequel le flavonoïde est choisi dans le groupe constitué de silibinine, silandrine, 3,4-dihydroxyflavone, hespéridine (glycoside), naringine (naringénine glycoside), amentoflavone, rutine, eriodictyol-7-O-glucoside, quercitrine, kaempferol, pinostrobine et biochanine A.

6. Procédé selon la revendication 1 ou la revendication 2, dans lequel le flavonoïde est choisi dans le groupe constitué de (+)-catéchine, myricétine, gossypétine et lutéonine.

7. Procédé ou utilisation selon l'une quelconque des revendications précédentes, dans lequel la peroxydase est une peroxydase hémique non animale de la classe II (fungi) ou de la classe III (plantes et algues).

8. Procédé ou utilisation selon la revendication 7, dans lequel la peroxydase est obtenue dans le groupe constitué d'Arabidopsis thaliana, raifort, orge, arachide, soja, tabac, et navet (plantes), Chlorophyta spirogyra (algues vertes), Arthromyces ramosus et Corprinus cinereus (fungi).

9. Procédé ou utilisation selon l'une quelconque des revendications précédentes, dans lequel la composition de couleur pour cheveux comprend 0,01 - 10, de préférence 0,1 - 5 % en masse/masse de flavonoïde.

**10.** Procédé ou utilisation selon l'une quelconque des revendications précédentes, dans lequel la composition de couleur pour cheveux comprend 0,0001 - 3, de préférence 0,001 - 1, encore mieux 0,01 - 1 % en masse/masse de peroxyde d'hydrogène.

**11.** Procédé ou utilisation selon l'une quelconque des revendications précédentes, dans lequel la composition de couleur pour cheveux comprend 0,0001 - 5, de préférence 0,001 - 1 % en masse/masse de peroxydase.

**12.** Procédé ou utilisation selon l'une quelconque des revendications 1 à 10, dans lequel le générateur de peroxyde d'hydrogène comprend une oxydase générant du peroxyde d'hydrogène, un substrat et de l'oxygène.

**13.** Procédé ou utilisation selon l'une quelconque des revendications précédentes, dans lequel la composition de couleur pour cheveux comprend de plus un ion de métal approprié pour une coordination au flavonoïde ou au produit oxydant du flavonoïde, l'ion de métal choisi de préférence dans le groupe constitué de fer (II), fer (III), cuivre (I), cuivre (II), cuivre (III) et aluminium (III).

**14.** Procédé ou utilisation selon l'une quelconque des revendications précédentes, dans lequel la composition de couleur pour cheveux est une composition de shampoing ou une composition d'après-shampoing.

# Fig. 1

DSC of human hair fibres treated with (+)- catechin / horseradish peroxidase

# Fig. 2a

Effect of treating unbleached human hair with catechin / horseradish peroxidase

# Fig. 2b

Effect of treating bleached human hair with catechin / horseradish peroxidase

# Fig. 3a

Denaturation temp. of natural white hair after treatment with catechin (no enzyme)

# Fig. 3b

### DSC of natural white hair after treatment with catechin / soy bean peroxidase and catechin / laccase

# Fig. 4a

### Effect of treating unbleached hair with catechin +/- enzyme

# Fig. 4b

Effect of treating bleached hair with catechin +/- enzyme

# Fig. 5a

Effect of treating bleached hair with catechin / laccase, myricetin / horseradish peroxidase and gossypetin / horseradish peroxidase

# Fig. 5b

Effect of treating bleached hair with catechin / laccase and luteolin / laccase

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5770418 A
- US 20040261198 A
- WO 2010130526 A
- WO 2010130510 A
- WO 9206154 A
- US 5194639 A
- WO 9631188 A
- EP 0530974 A
- US 4009256 A
- WO 9522311 A
- US 3962418 A
- US 3958581 A

**Non-patent literature cited in the description**

- **WORTMANN et al.** *J. Cosmet. Sci.,* 2002, vol. 53, 219-228